# EUROPEAN PATENT SPECIFICATION

(11) **EP 0 553 494 B1**
(45) Date of publication and mention of the grant of the patent: **04.10.2001**
(21) Application number: 92122084.4
(22) Date of filing: 29.12.1992
(51) Int. Cl.: C12N 15/21, C07K 14/56

(54) **Recombinant gene coding for human alpha interferon and expression vector thereof, ETC.**
Rekombinanter Gen kodierend für Alpha Interferon und Expressionsvector dafür
Gène recombinant codant pour l'interferon alpha humain et son vecteur d'expression

(30) Priority: 31.12.1991 KR 2587891; 31.12.1991 KR 2587991; 28.01.1992 KR 115592
(43) Date of publication of application: 04.08.1993
(60) Divisional application: 01101958.5
(73) Proprietor: LUCKY LTD., Seoul 150-721 (KR)
(72) Inventor: Cho, Joong Myung, Seoul 134-080 (KR); Park, Young Woo, Daejeon 305-340 (KR); Park, Soon Jae, Yuseong-gu, Daejeon 305-340 (KR); Bae, Tae Ok, Daejeon 300-120 (KR); Chang, Ho Jin, Yuseong-gu, Daejeon 305-340 (KR)
(74) Representative: von Samson-Himmelstjerna, Friedrich R., Dipl.-Phys.

(56) References cited:
- EP-A- 0 100 561
- EP-A- 0 128 467
- EP-A- 0 136 694
- EP-A- 0 156 373
- EP-A- 0 196 146
- EP-A- 0 360 937
- US-A- 4 751 078

## Description

### Field of the Invention

The present invention relates to a recombinant gene encoding human alpha interferon and the expression thereof in yeast. More particularly, the present invention relates to a recombinant gene encoding human alpha interferon useful for the mass production of human alpha interferon in yeast, an expression vector thereof, yeast cells transformed with said expression vector, a process for producing human alpha interferon by employing said yeast transformants, and a process for purifying said alpha interferon produced in yeast cells in a high yield.

### Description of the Prior Art

Interferon, a cytokin, is a protein with antiviral activities produced by animal cells in response to a viral infection. Interferons are classified on the basis of their origin: alpha interferon(IFN-α), also known as leucocyte interferon, is mostly produced by B lymphocytes, null lymphocytes or macrophages; beta interferon(IFN-β), also known as fibroblastic interferon, is mostly produced by fibroblasts or epidermal cells; and gamma interferon(IFN- ), also known as immune interferon, is mostly produced by T lymphocytes with macrophages(Stanton, G. J. et al., Tex. Rep. Biol. Med. 41, 84-88(1981); Kirchner, H. et al., Tex Rep. Biol. Med. 41, 89-93(1981)).

Hitherto, 20 or more types of alpha interferon genes have been identified; and most of them encode 165 or 166 amino acids.

The first alpha interferon used in a clinical test was one produced in buffy coat leukocyte stimulated by Sendai virus; and its purity was no more than about 1%(Cantell, K. and Hirvonen, Tex. Rep. Biol. Med. 35, 138-144(1977)).

Recently, human alpha interferons manufactured by using a recombinant DNA technology have been clinically tested. As a result, it has been reported that they are effective for the treatment of a number of cancerous diseases, particularly, bladder cancer(Torti, F. M. et al., J. Clin, Onco. 3, 506-512(1985)) and kindey cancer(Vugrin, D. et al., Cancer Treat. Rep. 69, 817-820(1985)), and, further, for the treatment of hepatitis C(Davis, G. G. et al., N. Engl. J. Med. 321, 1501-1506(1989); Bisceglie, A. D. et al., N. Engl. J. Med. 321. 1506-1510 (1989)).

The production of human alpha interferons using a recombinant DNA technology has generally been restricted to those processes employing E. coli as the host cell(Nagata, S. et al., Nature 284, 316-320(1980); Goeddel, D. V. et al., Nature 287, 411-416(1980); Streuli, M. et al., Science 209, 1343-1347(1980); Goeddel, D. V. et al., NAR 8, 4057-4074(1980); Dreynck, R. et al., Nature 287, 193-197(1980)). Alpha interferons so produced may be not identical to the natural one, since E. coli cannot glycosylate proteins produced therein; and E. coli native proteins such as endotoxin may remain in the alpha interferons even after its purification.

In this context, yeasts have several advantages over E. coli as a host cell for producing alpha interferons: that is, the mechanism of glycosylation in yeast is similiar to that in animal cells; and, further, yeasts do not produce any substance harmful to human, as borne out by the fact that yeasts have been used in the food processing industry for several centries.

Nevertheless, when interferons are prepared by employing a recombinant DNA technology in yeast cells, it is always critical to purify the interferons from yeast native substances for clinical use.

The alpha interferons produced in E. coli or yeast cells have been purified in high purity using reverse-phase high performance liquid chromatography(Rubinstein, M. et al., Arch. Biochem. Biophys. 210, 307-318(1981)) or monoclonal antibody affinity chromatography(Berg, K. and Heron, I., Methods Enzymol. 78, 487-499(1981)).

Needless to say, to be clinically useful, the bioactivity of interferons is important. In this connection, natural alpha interferon has two disulfide bonds, i.e., one between two cysteins which are the 1st and the 98th amino acids and the other between another pair of cysteins which are the 29th and the 138th amino acids. However, most alpha interferons produced by using E. coli or yeast exist as a reduced form not having said disulfide bonds or as an incompletely oxidized form having only one of said disulfide bonds, all of which are denatured forms. Further, they often form oligomers such as dimers by bonding between themselves during a purification process.

Therefore, many efforts have been made to obtain alpha interferon in an active form as well in a high degree of purity.

For example, Korean Patent Application No. 90-13450 discloses a method comprising the steps of: dissolving alpha interferon in a denatured form purified from a host cell by employing a conventional method in a solution containing a reducing agent such as β-mercaptoethanol and dithiothreitol; refolding said reduced alpha interferon and then isolating the refolded alpha interferon by an anion exchange chromatography to obtain alpha interferon with a high purity and activity. However, this method has a defect in that the refolded but non-active alpha interferon proteins, e.g., imcompletely oxidized ones or dimers still remain in the final product.

If a cation exchange chromatography is additionally employed, said non-active alpha interferon proteins can be removed. However, such removal will not be helpful for increasing the yield of active alpha interferon.

EP-A-0 128 467 desribes a DNA sequence for rINF-α, wherein at least one triplet encoding a cystein has been replaced by a triplet encoding an amino acid incapable of forming an intermolecular disulfide bond. EP-A-0 100 561 discloses the expression of unmodified human interferon in yeast.

### Summary of the Invention

It is an object of the present invention to provide a recombinant gene(rhIFN-α gene) encoding human alpha interferon for an efficient expression thereof in yeast.

It is another object of the present invention to provide an expression vector comprising said rhIFN-α gene and yeast cells transformed with the expression vector.

It is a further object of the present invention to provide a process for producing human alpha interferon(hIFN-α) by employing the yeast transformants.

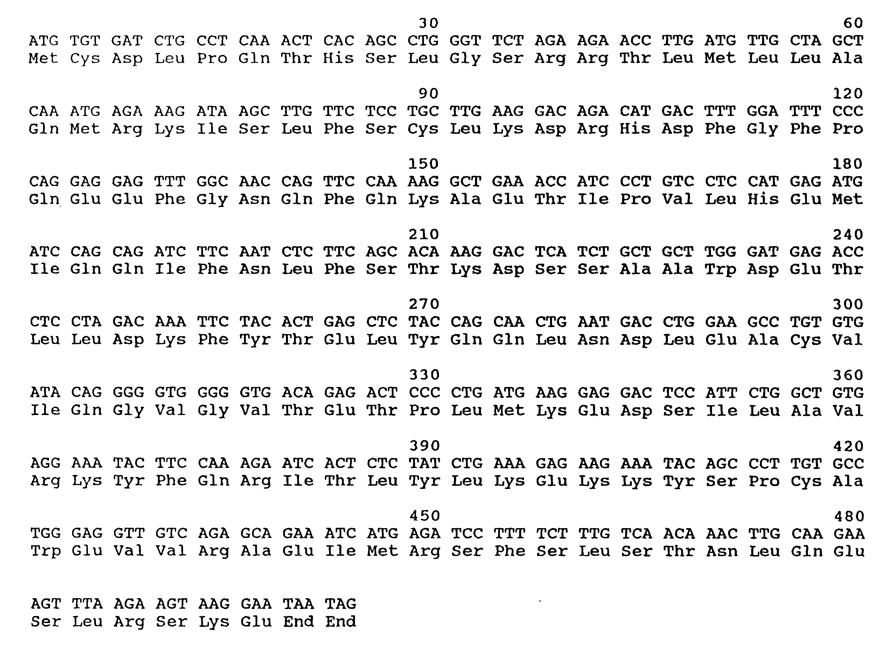

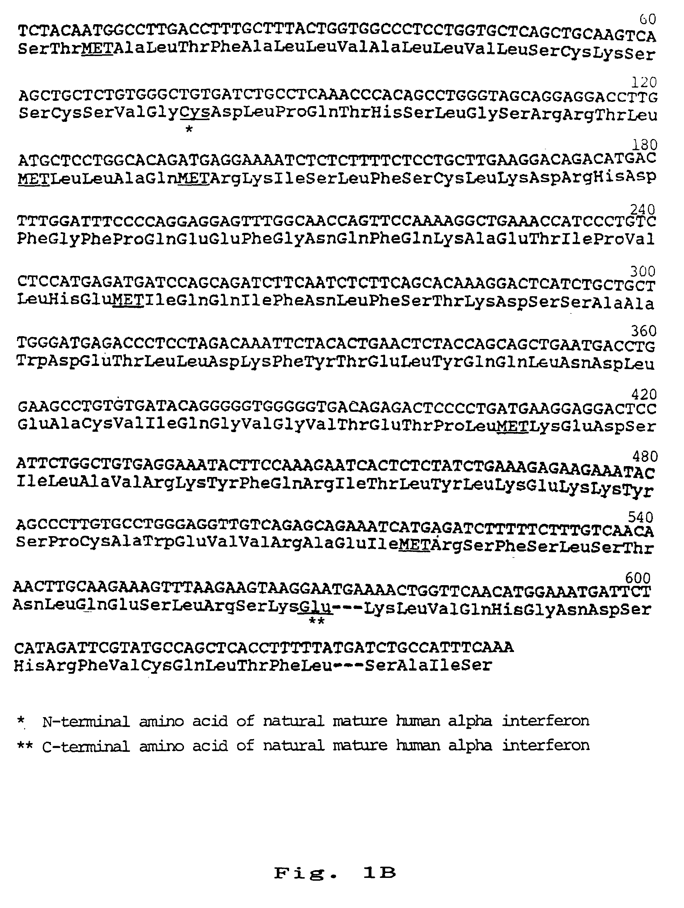

Accordingly, in accordance with one aspect of the present invention, there is provided with an rhIFN-α gene having a nucleotide sequence shown in Fig. 1 which is designed to produce mature hIFN-α in yeast.

In accordance with another aspect of the present invention, there is provided with an expression vector operable in yeast and comprising said rhIFN-α gene and yeast cells transformed with said vector.

In accordance with a further aspect of the present invention, there is provided with a process for producing hIFN-α in yeast comprising culturing the yeast tansformants under a culture condition appropriate for the expression of the rhIFN-α gene.

### Brief Description of the Drawings

Fig. 1A and 1B show the nucleotide sequence of an rhIFN-α gene of the present invention and the amino acid sequence encoded therein and the nucleotide sequence of natuaral hIFN-α cDNA prepared by employing an isolated human mRNA and the amino acid sequence encoded therein, respectively;
Fig. 2 represents the nucleotide sequence of the promoter AG885;
Fig. 3A and 3B describe the strategies for constructing two expression vectors containing the rhIFN-α gene;
Fig. 4A and 4B disclose the results of electrophoresis of cell extracts of the yeast transformants cultured under the condition appropriate for he expression of the rhIFN-α gene;
Fig. 5 depicts the result of CM-Sepharose column chromatography of the protein sample from the yeast transformants;
Fig. 6 presents the result of non-reducing sodium dodecyl sulfate-polyacrylamide gel electrophoresis for the dimers obtained through the use of CM-Sepharose column chromatography and the monomers converted from the dimers using a redox agant; and
Fig. 7 offers the result of C18 reverse-phase high performance liquid chromatography for the monomers obtained through the use of CM-Sepharose column chromatography and the monomers converted from the dimers using a redox agent.

### Detailed Description of the Preferred Embodiments

All references cited herein are hereby incorporated in their entirety by reference.

As used herein, the following terms shall have the following meanings:

The term "active form of human alpha interferon" or "active hIFN-α" refers to a monomeric human alpha interferon having two disulfide bonds, i.e., one between the two cysteins which are the 1st and the 98th amino acids and the other between the two cysteins which are 29th and the 138th amino acids.

The term "inactive form of human alpha interferon" or "inactive hIFN-α" refers to all human alpha interferons not having the two disulfide bonds described above, including those in an oligomeric form, incompletely oxidized form with one disulfide bond, and with incorrectly bonded disulfide bond.

All sequenced yeast genes, especially highly expressed genes, show a distinct preference for 25 codons among the 61 possible condons; and the degree of bias for these 25 preferred codons in each gene is correlated with the level of its mRNA in the cell(Bennetzent J. L. and Hall B. D., The Journal of Bio. Chem. 257, 3026-3031(1982)). Genes which are strongly expressed are more biased than genes with a lower level of expression.

Further, it is generally known that the efficiency of transcription and translation of a gene, which is an important factor for the expression of a gene, decreases if a secondary structure of mRNA exists.

On the basis of the above facts, the rhIFN-α gene of the present invention was designed to have yeast-preferred codons and to minimize the formation of a secondary mRNA structure by substituting the region corresponding to the 80 base pairs at the 5'-end region of hIFN-α cDNA prepared by employing mRNA isolated from human cells with a new one, so as to increase the transcription and translation efficiency of the rhIFN-α gene in a yeast cell.

Further, for the convenience of the gene manipulation, the recognition sites of four different restriction endonucleases were introduced to the rhIFN-α gene: Xba I site(5'-TCTAGA-3') is located at the codons for the 12th and 13th amino acids, Ser and Arg; Hind III site(5'-AAGCTT-3') was located at the codons for the 25th to the 27th amino acids, Ile, Ser and Leu; Bal II(5'-AGATCT-3') was located at the codons for the 63rd to the 65th amino acids, Glu, Ile and Phe; and Sac I(5'-GAGCTC-3') was located at the codons for the 88th and the 89th amino acids, Glu and Leu.

An initiation codon, ATG, is introduced just before the codon for the N-terminal amino acid of the mature alpha interferon, Cys; and two termination codons 5'-TAA-3' and 5'-TAG-3' were provided just after the codon for the C-terminal(166th) amino acid, Glu. For the convenience of cloning, an appropriate recognition site(s) of restriction endonuclease may be introduced to the position next to the termination codons and/or at the front of the initiation codon ATG.

The rhIFN-α gene of the present invention prepared to meet the above requirements has a nucleotide sequence as described in Fig. 1A. Fig. 1A shows also the amino acid sequence of the polypeptide encoded in said gene.

The expression vector of the present invention to express said rhIFN-α gene may be prepared by employing various expression systems operable in yeast.

In accordance with a preferred embodiment, there is provided an expression vector prepared by cloning the rhIFN-α gene in a vector comprising a GAP promoter. More specifically, the expression vector is prepared by using a plasmid pYLBC-GAP-HGH, which contains the promoter GAP, as described in the Examples given below, and named Luck-α-IFN-1Y.

In accordance with another preferred embodiment, there is provided an expression vector prepared by cloning the rhIFN-α gene in a vector comprising a promoter AG885 designed in accordance with the present invention, as follows:

Alcohol dehydrogenase 2(ADH2) of Saccharomyces cerevisiae is controlled by the presence of alcohol and glucose. It has been reported that, when glucose is exhausted in the medium, expression of the ADH2 gene increases about 200 times since the upstream activation sequence(UAS) comprising 22 base pairs is stimulated by the binding of ADR1-activating protein thereto (Yu et al., Molecular and Cellular Biology 9, 34-42(1989)). On the basis of the above, the present inventors have developed a novel promoter for gene expression in yeasts, which comprises 200 base pairs of polynucleotide having the DNA sequence substantially identical to the TATA box of GAPDH and 90 base pairs of polynucleotide having the DNA sequence substantially identical to the upstream activation sequence of ADH2; and named it promoter AG885.

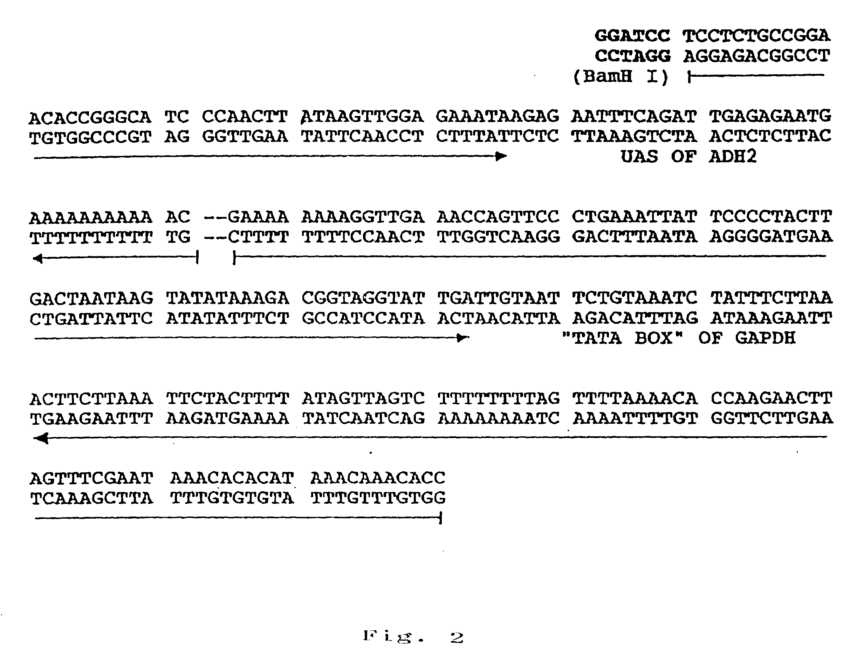

The nucleotide sequence of the promoter AG885 is shown in Fig. 2.

The expression vector of the rhIFN-α gene employing the promoter AG885 is prepared as described in the Examples given below, and named pYLBC-AG885-α-IFN.

The expression vector of the present invention, particularly, pYLBC-AG885-α-IFN, gives a high yield of hIFN-α and at least about 10% of the total yeast protein.

The Saccharomyces cerevisiae transformed with the vector Luck-α-IFN-1Y was deposited with Korean Culture Center of Microorganism on December 12, 1990 with the accession number of KFCC 10715, and conversion of the original deposit to a deposit under the terms of the Budapest Treaty on the International Recognition of the Deposit of Microorganisms for the Purpose of Patent Procedure was requested on December 12, 1992 and the accession number thereof is KCCM 10019. The Saccharomyces cerevisiae transformed with the vector pYLBC-AG885-α-IFN was deposited with Korean Collection for Type Culture on December 17, 1991 with the accession number of KCTC 0028BP, under the terms of the Buadpest Treaty.

A yeast cell transformed with the expression vector containing the rhIFN-α gene is then cultured under the condition capable of expressing the rhIFN-α gene, which is determined according to the characteristics of the vector and the host yeast cell.

The hIFN-α produced in the yeast transformant may be isolated and purified by a combined use of conventional methods, e.g., cell disruption, centrifugation, dialysis, salting out, chromatography, gel filtration, electrophoresis and electroelution; thereafter, hIFN-α having the same biological activity as the natural one is obtained in a high yield by the following method: dissolving the hIFN-α isolated from yeast cells in a solution containing a reducing agent; refolding the reduced hIFN-α under a refolding condition; subjecting the solution containing the refolded hIFN-α to an anion exchange chromatography to elute the refolded hIFN-α as an eluate; subjecting the eluate to a cation exchange chromatography to separate inactive hIFN-α from the active hIFN-α; and treating the inactive hIFN-α with a redox agent containing oxidized thiol residues and reduced thiol residues to convert it to the active hIFN-α.

The inactive form of hIFN-α can be converted to the active hIFN-α by the treatment thereof with a redox agent, particularly, a redox agent containing oxidized thiol residues and reduced thiol residues in a suitable ratio.

In said redox agent, the concentration of the reduced thiol residues is preferably about 1 to 10 mM and the concentration of the oxidized thiol residues is preferably about 0.1 to 1 mM. Said redox agent includes, for example, those comprising cysteine and cystine and also those comprising oxidized glutathione and reduced glutathione.

In accordance with the above purification process, the polypeptide biologically idential to human alpha interferon can be purified in a high yield.

The following Examples are intended to specifically exemplify the present invention without limiting its scope; and the experimental methods used in the Examples are practised in accordance with the Reference Examples given hereinbelow.

Unless otherwise specified, percentages given below for solids in solid mixtures, liquids in liquids and solids in liquids are on a wt/wt, vol/vol and wt/vol basis, respectively.

### Reference Example 1: Digestion of DNA with Restriction Endonuclease

The restriction endonucleases and reaction buffers used herein were purchased from NEB(New England Biolabs, U.S.A.).

The reaction was generally carried out in a sterilized Eppendorf tube with a reaction volume ranging from 50 to 100*µ*l, at a temperature of 37°C for 1 to 2 hours. Thereafter, the reaction mixture was heat-treated at 65°C for 15 minutes (or extracted with phenol and precipitated with ethanol in the case of a heat-resistant endonuclease) to inactivate the restriction endonuclease.
10 x reaction buffer for the reaction of a restriction endonuclease had the following composition:
10 x NEB reaction buffer 1: 100mM bis Tris propane-HCl, 100mM MgCl₂, 10mM dithiothreitol(DTT), pH 7.0;
10 x NEB reaction buffer 2: 100mM Tris-HCl, 100mM MgCl₂, 500mM NaCl, 10mM DTT, pH 7.0;
10 x NEB reaction buffer 3: 100mM Tris-HCl, 100mM MgCl₂, 10mM DTT, pH 7.0; and
10 x NEB reaction buffer 4: 200mM Tris-acetate, 100mM magnesium acetate, 500mM potassium acetate, 10mM DTT, pH 7.0.

### Reference Example 2: Phenol Extraction and Ethanol Precipitation

After the completion of the enzyme reaction, the reaction mixture was extracted with phenol for the purpose of inactivating the enzyme or recovering the DNA in the reaction mixture, wherein phenol preequilibrated with a buffer containing 10mM Tris-HCl(pH 8.0) and 1mM EDTA was used. Phenol extraction was carried out by mixing equal volumes of a sample and the phenol with vigorous shaking; centrifuging the mixture at 15,000rpm for 5 minutes; and transferring the aqueous layer into a new tube. The above procedure was repeated two or three times.

The aqueous layer was, then, extracted with an equal volume of chloroform solution(chloroform : isoamyl alcohol = 24:1) and the aqueous layer was separated again; 0.1 volume of 3M sodium acetate and 2.5 volumes of ethanol were added thereto; and, the mixture was centrifuged at 15,000rpm and 4°C for 20 minutes after having left it at -70°C for 30 minutes or at -20°C for 12 hours, to recover the nucleic acid.

### Reference Example 3: Ligation Reaction

Ligation of DNA was carried out by employing T₄ DNA ligase and 10 x ligation reaction buffer(0.5M Tris-HCl, 0.1M MgCl₂, 0.2M DTT, 10mM ATP, 0.5mg/ml bovine serum albumin (BSA)) purchased from NEB. The reaction volume was generally 20*µ*l, and 10 units of T₄ ligase was used for the ligation of the cohesive ends of DNA while 100 units of T₄ ligase was used for the ligation of blunt ended DNAs.

The reaction was carried out at 16°C for 5 hours or at 4°C for over 14 hours; and, after the reaction was completed, the reaction mixture was heated at 65°C for 15 minutes to inactivate the T₄ DNA ligase.

### Reference Example 4: Transformation of E. coli

E. coli strains used for the following Examples include E. coli HB101(ATCC 33694), E. coli W3110(ATCC 27325), and E. coli JM105(ATCC 47016).

The host cells were incubated in 100ml of LB liquid medium(1% Bacto tryptone, 0.5% Bacto yeast extract, 0.5% NaCl) at 37°C until the O.D. (optical density) value at 650nm reached the range from 0.25 to 0.5. The cell culture was isolated by the centrifugation and then washed with 50ml of 0.1M magnesium chloride(MgCl₂) solution. The cell pellet obtained by the centrifugation was added with 50ml of solution of 0.1M CaCl₂ and 0.05M MgCl₂ and then stood in an ice bath for 30 minutes. The cells were isolated by centrifugation and then suspended in 5ml of the solution of 0.1M CaCl₂ and 0.05M MgCl₂. The reagent and materials used above had been cooled to 0°C just before the use.

To 0.2ml of cell suspension obtained above was added 10*µ*l of a ligation reaction mixture; and the reaction mixture was stood at 0°C for 40 minutes and then heated at 42°C for 1 minute. 2.0ml of LB liquid medium was added thereto; and the cells so treated were incubated at 37°C for 1 hour. The culture was then applied onto a Petri dish containing LB solid medium provided with an appropriate antibiotic such as ampicillin and incubated at 37°C overnight to obtain transformant colonies.

In the case of transformation of E. coli with M13 vector, M13 vector DNA(ligated with a gene) was added with 100*µ*l of JM105 cell suspension, 15*µ*l of 10mM isopropyl β-thioglactopyranoside, 25*µ*l of 4% X-Gal and 3ml of 2 x YT soft solid medium(1% NaCl, 1% yeast extract, 1.6% Bactotryptone, 0.7% Bacto agar) preheated to 38°C. The mixture was stirred and applied onto 2x YT solid medium(1% NaCl, 1% yeast extract, 1.6% Bacto tryptone, 1.5% Bacto agar) and incubated at 37°C overnight to obtain plaques.

### Reference Example 5: Synthesis of Oligonucleotides

Oligonucleotides were synthesized by employing a DNA synthesizer(Applied Biosystems Inc., 380B, U.S.A.) of automatic solid phase phosphoamidite chemistry.

The synthesized oligonucleotides were purified by using a denaturing polyacrylamide gel(2M urea, 12% acrylamide and bis acrylamide(29:1), 50mM Tris, 50mM boric acid, 1mM EDTA-Na₂) electrophoresis and SEP-PAK(Waters Inc., U.S.A) column chromatography using a mixture of acetonitrile and water (50:50) as an eluent; and the amount was determined by measuring the O.D. value at 260nm.

### Reference Example 6: Polymerase Chain Reaction(PCR)

To a mixture of 10 to 100ng of template DNA, 10*µ* l of 10x Taq polymerase reaction buffer(10mM Tris-HCl, 500mM KCl, 15mM MgCl₂, 0.1%(w/v) gelatin, pH 8.3), 10*µ*l of a mixture of dNTP (each of dGTP, dATP, dTTP and dCTP is 1.25mM), 2*µ*g of each primer(generally, 2 primers were used for the reaction, and in case 3 primers were used, the primer located in the middle was used in an amount of 0.02*µ*g), and 0.5*µ*l of Ampli Taq DNA polymerase(Perkin Elmer Cetus, U.S.A.) was added with distilled water in an amount to make a total volume of 100*µ*l; and 50*µ*l of mineral oil was added thereto to protect the reaction mixture from evaporation.

The PCR was carried out by using a thermal cycler(Perkin Elmer Cetus, U.S.A.); and the thermal cycle was programmed to repeat 25 times or more, the cycle being: 95°C for 1 minute → 55°C for 1 minute → 72°C for 2 minutes; and, finally, the reaction was carried out at 72°C for 10 minutes.

After the reaction was completed, the mixture was extracted with phenol and the PCR products were recovered by precipitation with ethanol; and, the precipitate was dissolved in 20*µ*l of TE buffer solution(lOmM Tris-HCl, 1mM EDTA, pH 7.5).

### Example 1 : Preparation of human alpha interferon cDNA

### (1-A): Preparation of mRNA from human cell

### <Step 1>: Extraction of total RNA from human cell line KG-1

Human cell line KG-1(ATCC CCL246) was incubated in RPMI164 medium(Gibco, Cat. #430-3400EB, Grand Island, New York 14072 U.S.A.) for 2 to 3 days; and the medium was removed off by the centrifugation(750 x g, 10min). The cell pellet(about 5 x 10⁷ cells) was suspended in fresh RPMI164 medium in the concentration of about 10⁶ cells/ml. To the suspension was added 1/200 volume of 200mM sodium butyrate and the mixture was then incubated for 48 hours at 37°C. The culture was centrifuged again and the cell pellet was isolated and suspended in fresh RPMI164 medium.

In order to induce the production of hIFN-α mRNA in the cell so obtained, NCD virus(New Castle disease virus, ATCC VR0107) was added to the cell suspension, and the mixture was then incubated at 37°C for 12 to 20 hours. About 5 x 10⁷ cells induced as above were precipitated by the centrifugation (750 x g, 10min) and the cell pellet was lysed in 10ml RNAzol B(Cinna/Biotecs, U.S.A.). To the cell lysate was added 1ml of chloroform and the mixture was vigorously shaken for 15 seconds and stood in an ice bath for 5 minutes. The mixture was centrifuged at 12000 x g, 4°C for 15 minutes to separate water/chloroform phases. The upper phase was taken and added with an equal volume of isopropanol.

The mixture was stood at 4°C for 15 minutes and centrifuged at 1200 x g, 4°C for 15 minutes to precipitate RNA. The RNA precipitate was suspended in 200*µ*l of distilled water treated with diethylpyrocarbonate(DEPC); and the mRNA tailed with Poly(A)⁺(Poly(A⁺) mRNA) was immediately isolated therefrom.

### <Step 2>: Isolation of Poly(A)⁺ mRNA

The isolation of Poly(A)⁺ mRNA was carried out using an mRNA Isolation Kit(Stratagene Inc., Cat. #200348, U.S.A.), as follows.

The sample (200*µ*l) obtained in <Step 1> above was heated at 65°C for 5 minutes, and then added with 20*µ*l of 10 x sample buffer (10mM Tris-HCl, 1mM EDTA, 5.0M NaCl, pH 7.5) on ice. Thereafter, the mixture was immediately loaded on an oligo-d(T) cellulose column at the rate of 1 drop/2 seconds. The oligo-d(T) cellulose column was then washed twice by loading with each 200*µ*l of high salt buffer(10mM Tris-HCl, pH 7.5, 1mM EDTA, 0.5M NaCl) thereon at the rate of 1 drop/2 seconds. Poly(A)⁺ mRNA was eluted with 200*µ*l of low salt buffer(10mM Tris-HCl, pH 7.5, 1mM EDTA, 0.1M NaCl). The eluted sample was subjected to ethanol precipitation and then dissolved in 100*µ*l of TE solution.

### (1-B): Preparation of cDNA library

### <Step 1>: Preparation of cDNA

For the preparation of cDNA from the Poly(A)⁺ mRNA, Zap-cDNA Synthesis Kit (Stratagene Inc., USA, Cat. #200400) was used. The Poly(A)⁺ mRNA was used as a template, and oligo-d(T) primer having the nucleotide sequence of 5'-GAGAGAGAGAGAGAGAGAGAGAACTAGTCTCG AG(T)₁₈-3' was used.

A first strand of cDNA was prepared as follows. 18*µ*l of mRNA solution prepared in Example(1-A) was mixed with 2*µ*l of 0.1M CH₃HgOH, and the mixture was stood for 10 minutes at room temperature to unfold a secondary structure of RNA. To the resultant mixture was added 2*µ*l of 1M β-mercaptoethanol, which mixture was kept for 5 minutes at room temperature. Thereto were added 5*µ* l of reverse transcriptase reaction buffer solution (500mM Tris-HCl, pH 8.3, 750mM KCl, 30mM MgCl₂, 10mM DTT), 2.5*µ*l of 10mM each of dATP, dGTP, dTTP and 5-methyl-dCTP, 2*µ*l of oligo-d(T) primer (1.4*µ*g/*µ*l), 15*µ*l of distilled water treated with DEPC and 1.0*µ*l of RNase inhibitor(1 unit/*µ*l, Promega Inc., USA), in the above order; the mixture was stood for 10 minutes at room temperature to bind the primers to the template; and then 2.5*µ*l of Superscript RNase H⁻ reverse transcriptase(180 units/*µ*l, BRL Inc., Cat. No. 8853SA) was added thereto. The reaction mixture was incubated for 1 hour at 37°C to synthesize the first strand of cDNA.

A second strand of cDNA was prepared as follows. To 45*µ*l of the first strand solution obtained above were added 40*µ*l of 10 x second strand buffer solution(188mM Tris-HCl, pH 6.9, 906mM KCl, 46mM MgCl₂, 1.5mM β-NAD(nicotinamide adenine dinucleotide), 10mM(NH₄)₂SO₄), 6.0*µ*l of 10mM each of dATP, dCTP, dTTP and dGTP and 298*µ*l of distilled water, in the above order; and 1.0*µ*l of RNase H(4units/*µ*l) and 10.0*µ*l of DNA polymerase I(11 units/*µ*l) were then dropped along the wall of the test tube. After instant mixing, the reaction mixture was then incubated for 2.5 hours at 16°C.

The resultant mixture was then extracted with an equal volume of phenol-chloroform mixture(1:1(v/v), phenol being already saturated with 0.5M Tris-HCl(pH 7.5) and 0.1%(v/v) *β*-mercaptoethanol), 3 times. The upper aqueous phase was taken and mixed with 0.1 volume of 3M sodium acetate and 2 volumes of 100% ethanol. The mixture was stood at -20°C overnight and centrifuged at 12,000 x g, 4°C for 20 minutes to obtain the cDNA precipitate.

### <Step 2>: Preparation of cDNA library

In order to make the double stranded cDNA into a blunt ended one, the cDNA precipitate prepared in the above <Step 1> was dissolved in 43.5*µ*l of distilled water. 39*µ*l of the cDNA solution was taken and then mixed with 5.0*µ*l of T4 DNA polymerase reaction solution(670mM Tris-HCl, pH 8.8, 166mM (NH₄)₂SO₄, 67mM MgCl₂, 100mM β-mercaptoethanol, 67*µ*M EDTA), 2.5*µ*l of 2.5mM dNTP's mixture and 3.5*µ*l of T4 DNA polymerase (2.9 units/*µ* l). The reaction mixture was stood for 30 minutes at 37°C and the product was extracted with the phenol-chloroform mixture and precipitated with ethanol in the same manner as in the above <Step 1>.

In order to provide recognition sites for restriction endonucleases Eco RI and Xho I at the 5'-end and the 3'-end, respectively, the blunt-ended double stranded cDNA prepared above was treated as follows: To the precipitate containing blunt-ended cDNA obtained above were added 7.0*µ*l of Eco RI adaptor(Stratagene Inc., Zap-cDNA Synthesis Kit Cat. No. 200400, U.S.A.), 1.0*µ*l of 10 x ligation buffer solution (500mM Tris-HCl, pH 7.8, 100mM MgCl₂, 200mM DTT, 500*µ*g/ml BSA), 1.0*µ*l of T4 DNA ligase (1000 units/µl) and 1.0*µ*l of 10mM ATP. The resultant mixture and stood overnight at 4°C and then heated at 70°C for 10 minutes to inactivate the ligase.

The product so obtained was extracted with the phencl-chloroform mixture and precipitated with ethanol in the same manner as above, and then dissolved in 16*µ*l of TE buffer solution.

To the resultant solution were added 2*µ*l of 10x buffer solution(0.5M NaCl, 0.5M Tris-HCl, 50mM MgCl₂, 5mM DTT, pH 7.9) and 1*µ*l of each of Eco RI and Xho I(New England Biolabs Inc., U.S.A.), and the reaction mixture was then stood for 10 minutes at 37°C to digest the cDNA partially. The cDNA fragments were extracted with the phenol-chloroform mixture and precipitated with ethanol in the same manner as above, and then dissolved in 10*µ*l of TE buffer solution.

The cDNA fragment so obtained was cloned into vector UNI-ZAPXR as follows. To 10*µ* l of the cDNA fragments solution digestd with Eco RI-Xho I were added 2.0*µ*l of 10x ligation buffer solution, 2.0*µ*l of 10mM ATP, 4.0*µ*l of vector UNI-ZAPXR solution(1*µ*g/*µ*l, Stratagene Inc., U.S.A.) already digested with Eco RI/Xho I and 2.0*µ*l of T4 DNA ligase(4 Weiss units/*µ*l); and the reaction mixture was then incubated for 10 hours at 16°C.

### <Step 3>: In vitro packaging of the vector containing cDNA into phage and amplification of the cDNA library

In order to package the ligated DNA into a phage, 10*µ*l of the final solution obtained in the above <Step 2> was added to Gigapack II Gold Packaging Extract (Stratagene Inc., U.S.A.) and the reaction mixture was stood for 2 hours at room temperature. To the resultant mixture were added 500*µ*l of a phage diluting solution(5.8g of NaCl, 2.0g of MgSO₄·7H₂O, 50ml of 1M Tris-HCl, pH 7.5, 5ml of 2% gelatin per liter) and 20*µ*l of chloroform (see Kretz et al., Nucl. Acid. Res. 17, 5409(1989)).

The infection and amplification thereof was carried out as follows. PLK-F'(Stratagene Inc., Zap-cDNA Synthesis Kit, Cat. No. 200400), E. coli merA⁻, merB⁻ strain, was cultured in LB medium until the O.D. value at 600nm(O.D.₆₀₀) reached 0.5. The cultured cells were precipitated and then dissolved in 10mM MgSO₄, adjusting the O.D.₆₀₀ value to 1.0. 600*µ*l of the solution was mixed with 20*µ*l of packaging mixture; the reaction mixture was then stood for 15 minutes at 37°C to allow the packaged phage particles to infect into E. coli. To the resultant E. coli was added 6.5ml of 0.7% NZY agar(7g of NZ amines, 5g of NaCl, 2g of MgSO₄·7H₂O, 5g of yeast extract, 7g of Bacto agar per liter) melted and kept to 48°C; and the mixture was applied onto a 150mm-diameter NZY agar plate(7g of NZ amines, 5g of NaCl, 2g of MgSO₄·7H₂O, 5g of yeast extract, 16g Bacto agar per liter) and then incubated for 5 to 8 hours at 37°C to generate the phage plaques. 10ml of the phage diluting solution was poured onto a plate; and the plate was shaken mildly for 15 hours at 4°C to dissolve the phages; and the resultant mixture was centrifuged at 4,000 x g to precipitate E. coil cells, which were then removed off. To the cDNA library solution so obtained was added 0.3% volume of chloroform; and the titer of the cDNA library was determined to be about 10¹⁰ to 10¹³ PFU(plaque forming units)/ml. 100% DMSO(dimethyl sulfoxide) was added thereto to make a concentration of 7%(v/v); and the cDNA library was kept at -70°C.

### (1-C): Screening of the cDNA library by immunoassay and determination of cDNA sequence

The cDNA library was screened by the immunoscreening method disclosed by Huynh, T. V. et al., DNA Cloning Techniques: A Practical Approach (D. M. Glover, ed.), pp49-78, IRL Press, Oxford(1985), to select the phages containing INF-α cDNA.

The cDNA library solution prepared in Example(1-B) was diluted to be 50,000 PFU per 150mm-diameter plate; the diluted cDNA library solution was mixed with 600*µ*l of E. coli XL-1 blue(Stratagene Inc., U.S.A., Zap-cDNA Synthesis Kit Cat. No. 200400) culture(O.D.₆₀₀ = 0.5) prepared by the same method as in <Step 2> of Example(1-B) and 6.5ml of 0.7% NYZ agar was added thereto. Each mixture was applied onto a 40 NZY agar plate and cultured for 12 hours at 37°C to produce 2 x 10⁶ phage plaques.

Thereafter, 132mm diameter nitrocellulose filters were dipped in a 10mM IPTG(isopropyl β-D-thiogalactopyranoside) solution and then blot-dried on Whatman 3MM filters. Each filter was placed above the agar in each plate; and incubated for 3.5 hours at 37°C. Each of the filters blotted with the phage plaques was then washed with 15ml of washing solution (10mM Tris-HCl, pH 8.0, 150mM NaCl, 0.05% Tween 20). To the filters was added 15ml of blocking solution(1% bovine serum albumin, 20mM Tris-HCl, pH 7.5, 150mM NaCl); and the resultant was incubated with gentle shaking for 1 hour at room temperature. Each of the filters was then washed 5 times with mild shaking by using 15ml of TBST buffer solution(20mM Tris-HCl, pH 7.5, 150mM NaCl, 0.05%(v/v) Tween-20) for 5 minutes at room temperature. The filters were put in 15ml of a solution prepared by 100-fold diluting the anti IFN-α antibody(Cat #853569; Boehringer Mannheim, U.S.A.) with TBS buffer solution(20mM Tris-HCl, pH 7.5, 150mM NaCl) containing 1%(w/v) FBS(fetal bovine serum) with mild shaking for 1 hour at room temperature; and then washed 5 times with mild shaking in TBST buffer solution for 5 minutes at room temperature, each time. Each of the filters was put in 15ml of a solution prepared by 2000-fold diluting biotinylated-goat anti-mouse IgG and avidin conjugated-alkaline phosphatease(Pierce Inc., U.S.A.) with TBS buffer solution containing 1%(w/v) FBS, with mild shaking for 1 hour at room temperature; and then washed 5 times with gentle shaking in 15ml of TBST buffer solution for 5 minutes at room temperature. Each of the filters was then blot-dried on a Whatman 3MM filter.

For the coloring reaction, each of the filters was reacted in 15ml of a coloring solution(100mM Tris-HCl, pH 9.5, 100mM NaCl, 5mM MgCl₂, 5mg nitro blue-tetrazolium, 2.5mg 5-bromo-4-chloro-3-indolyl phosphate) in a dark room at room temperature for 30 minutes. The purple-colored, positive phage plaques were confirmed with eyes, which were expected to express the cDNA encoding IFN-α. Each of the filters was washed with TBS buffer solution once; and a coloring stopping solution(20mM Tris-HCl, pH 2.9, 1mM EDTA) was added thereto to stop the coloring process. Each of the filters was dried at room temperature and then recorded on polaroid film.

Positive plaques were isolated; and incubated in 1ml of a phage diluting solution(10mM Tris-HCl, pH 7.5, 10mM MgCl₂) for 1 to 2 hours at room temperature. The above immunoscreening assay was repeated to obtain the colonies as a single phage plaque.

Each of the phage plaques confirmed as containing the gene encoding hIFN-α was placed into a sterilized microfuge tube containing 500*µ*l of SM buffer solution(5.8g of NaCl, 2.0g of MgSO₄, 50*µ*l of 1M Tris-HCl, pH 7.5, 5ml of 2% gelatin per liter); 20*µ*l of chloroform was added thereto; and then the content of the tube was cultured with shaking for 1 to 2 hours at room temperature. 200*µ*l(> 1 x 10⁵ phage particles) of the solution so obtained, 1*µ*l of helper phage R408(> 1 x 10⁶ PFU/ml, Stratagene Inc., U.S.A.) and 20*µ*l of E. coli XL-1 blue cell supension(O.D.₆₀₀ = 1.0) were mixed, and then the mixture was incubated at 37°C for 15 minutes. To the resultant culture was added 5ml of 2 x YT medium(10g of NaCl, 10g of yeast extract, 16g of Bacto-tryptone per liter), which was cultured with shaking for 3 hours at 37°C and then heated at 70°C for 20 minutes. The resultant culure was 100-fold diluted; and 200*µ*l of the diluted culture was mixed with 200*µ*l of E. coli XL1-Blue cell(O.D.₆₀₀ = 1.0). After incubating at 37°C for 1 hour, 100*µ*l of the resultant culture was applied onto LB plates containing ampicillin(50*µ*g/ml); and incubated at 37°C for 10 hours to obtain pBluescript phagemid colonies containing double stranded cDNA.

In order to prepare single stranded DNA, the pBluescript colonies obtained above were incubated in an LB agar medium containing antibiotic tetracycline(12.5*µ*g/ml) and screened to obtain the positive colonies again; and the positive single colonies so obtained were incubated in a tetracycline⁺ LB broth medium(2 to 3ml) overnight. The culture was then incubated in 0.3ml of super liquid medium(35g Bacto tryptone, 20g yeast extract, 5g NaCl, adjusted to pH 7.5 with NaOH) and cultured with shaking at 37°C. The culture was infected with helper phage R408 and the culturing thereof was carried out for 8 hours until O.D.₆₀₀ reached 0.3.

The isolation and purification of the recombinant phage nucleotides was carried out by the method proposed by Sambrook, J. et al. in Molecular Cloning, 1, 2.73-2.81, Cold Spring Harbor, N. Y. (1989).

The nucleotide sequences were determined using the purified single stranded recombinant pBluescript phagemid or the double stranded pBluescript phagemid as a template and using M13-20mer, primer T7, primer KS, primer SK or primer T3 (Stratagene Inc., USA) in accordance with Sanger's method (Proc. Natl. Acad. Sci. 74, 5405(1977)), which is shown in Fig. 1B with the amino acid sequences deduced from the nucleotide sequence.

### Example 2: Modification of the nucleotide sequence of hIFN-α cDNA by employing synthesized oligonucleotides

The following four oligonucleotides were designed and synthesized so that the expression of the hIFN-α cDNA could be optimized by substitution of the codons at the 5'-end of hIFN-α cDNA with the preferred codons for yeast without changing the amino acid sequence, and simultaneously, its capability of forming a secondary mRNA structure was removed so as to increase the expression efficiency of hIFN-α in yeast at the level of transcription and translation.

IFN1 : 5'-AAAACATGTGTGATCTGCCTCAAACTCACAGCCTGGGTTCTAGAAGAACCTT
XbaI site
GATGTTGCTAGCTCAAATGAGAAAGATAAGCTTGTTCTCCTG-3'
IFN2 : 5'-GTCATTCAGTTGCTGGTAGACCTCAGT-3'
IFN3 : 5'-ACTGAGCTCTACCAGCAACTGAATGAC-3'
IFN4 : 5'-GGGGTCGACTATTATTCCTTACTTCTTAAACTTTCTTGCAAGTTTGTTGAC
SalI site
AAAGAAAAGGATCTCATGA-3'

The PCR was carried out by using the above oligonucleotides. To a tube A were added 2*µ*g of IFN1 and 2*µ*g of IFN2; and to a tube B were added 2*µ*g of IFN3 and 2*µ*g of IFN4. To each of the tubes were added 100ng of DNA containing hIFN-α cDNA obtained in Example(1-C), 10*µ*l of 10 x Taq polymerase buffer, 10*µ*l of 10mM dNTP and 2.5 units of Taq polymerase; and distilled water was added thereto to adjust the total volume to 100*µ*l. After the first PCR was carried out in accordance with the method of Reference Example 6, the PCR products having about 250bp from each of tubes A and B(hereinafter referred to as "PCR product A and B", respectively) were isolated by precipitation with ethanol.

Thereafter, a second PCR was carried out by using PCR products A and B under the same condition as in the above. About 510 bp of nucleic acid fragment was obtained and named IFN-Y.

### Example 3: Cloning of rhIFN-α gene into vector M13mp18 and determination of nucleotide sequence

To 10*µ*l (about 2*µ*g of nucleic acid) of TE buffer solution containing IFN-Y obtained in Example 2 were added 3*µ*l of 10 x NEB buffer solution 3, 16*µ*l of distilled water and 1*µ*l of a restriction endonuclease SalI(20units/*µ*l); and the mixture was reacted over 1 hour at 37°C and subjected to electrophoresis on 5% polyacrylamide gel to isolate 510 bp of digested fragment. The isolated fragment was purified with Elutip-D(Schleicher & Schuell, U.S.A.). The purified fragment was named IFN-L and dissolved in 10*µ*l of distilled water before use for ligation.

On the other hand, double-stranded M13mp18 DNA(New England Biolabs, U.S.A.) was digested with restriction endonucleases SmaI and SalI and the fragment was isolated in a purified state. To the mixture of 2*µ*l of 10 x ligation buffer solution(660mM Tris-HCl, pH 7.6, 66mM MgCl₂, 100mM DTT), 2*µ*l of 100mM ATP, 2*µ*l(0.2*µ*g) of IFN-L, 1*µ*l(about 0.1*µ*g) of M13mp18 DNA digested with SmaI and SalI and 12*µ*l of distilled water was added 1*µ*l of T4 DNA ligase; and the resultant solution was reacted for 5 hours at 16°C. Thereafter, 10*µ* l of the ligating resultant was reacted with E. coli JM105(ATCC 47016) in accordance with the Hanahan method described in J. Mol. Biol. 116, 557(1983) for the transformation of E. coli cell.

The E. coli cell mixture thus treated was added with 7.5*µ*l of 0.2M IPTG, followed by mixing it with 3ml of 2 X YT soft agar medium(16g of Bacto tryptone, 10g of yeast extract, 5g of NaCl, 6g of Bacto agar per 1L) and 25*µ*l of 4% X-gal solution. The resultant mixture was spread on a Min A solid medium(10.5g of K₂HPO₄, 4.5g of KH₂PO₄, 1g of (NH₄)₂SO₄, 0.5g of Na-citrate, 12g of Bacto agar, 1ml of 20% MgSO₄, 0.5ml of 1% Vit. B and 10ml of glucose per 1L) and incubated at 37°C overnight.

A white plaque thus obtained was isolated and employed to obtain a single stranded nucleic acid in accordance with the method proposed by Sambrook, J. et al., in Molecular Cloning, Cold Spring Harbor, N.Y.(1989). The nucleotide sequence of the recombinant hIFN-α gene was determined in accordance with the dideoxy DNA sequencing method(Sanger, F. et al., P.N.A.S., 84 4767 (1987); rhIFN-α gene having the nucleotide sequence shown in Fig. 1A was selected; and the M13mp18 containing said nucleotide sequence was named M13mp18-IFN-α.

### Example 4: Preparation of expression vector

### (4-A): Preparation of vector Luck-α-IFN-1Y

About 2*µ*g of plasmid pYLBC-GAP-HGH(Korean Patent Laid-open No. 90-9975) was completely digested with the restriction endonucleases PstI and SalI in NEB buffer solution 3; and another 2*µ*g of said plasmid was completely digested with the restriction endonucleases PstI and Bst BI in NEB buffer solution 4. Each of the digested plasmids was passed through 0.7% agarose gel to isolate about 9.8Kb and 3.4Kb of fragments which were named fragments PL and PB.

About 5*µ*g of vector M13mp18-IFN-α containing an rhIFN-α gene obtained in Example 3 was completely digested with the restriction enzymes Xbal and SalI in NEB buffer solution 3 and was subjected to phenol-chloroform extraction and ethanol precipitation. The precipitated nucleic acid(hereinafter referred to as IFN-X/L) was dissolved in 10*µ*l of TE solution.

The ligation thereof was carried out by using the fragments obtained in the above and a synthesized linker

(5'-TCGAATAAACACACATAAACAAACACCATGTGTGATCTGCCTCAAACTCACAGCCT
3'-TATTTGTGTGTATTTGTTTGTGGTACACACTAGACGGAGTTTGAGTGTCGGA
-GGGTT-3'
-CCCAAGATC-5').

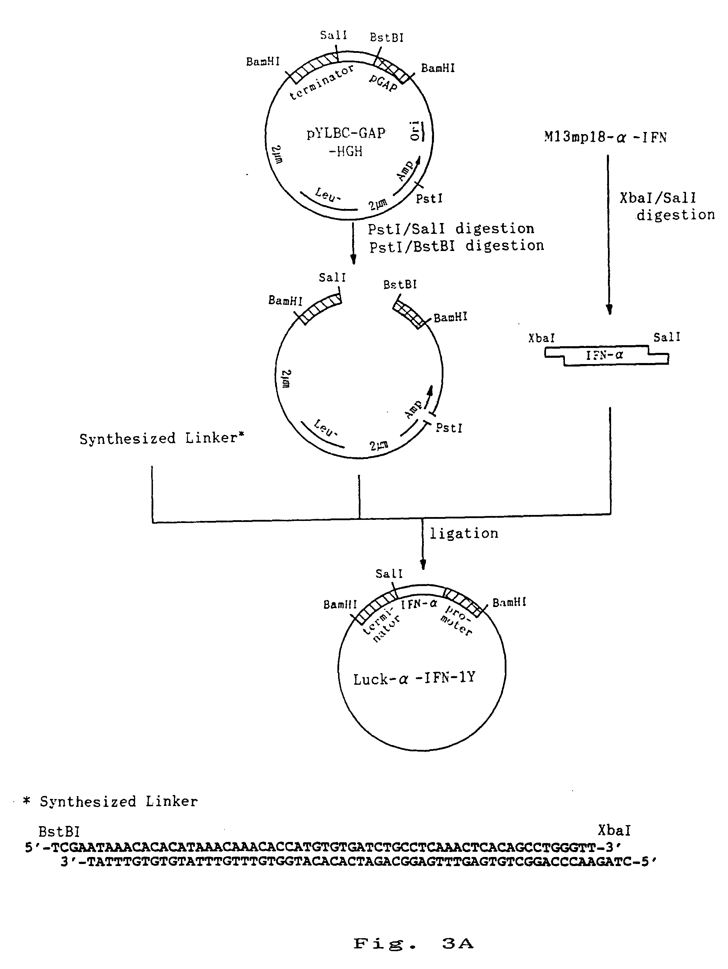

To a tube were added 200ng of fragment IFN-X/L, 50ng of the linker, 100ng of a fragment PL, 100ng of fragment PB, 2*µ*l of 10 x ligation solution and 10 units of T4 DNA ligase; and distilled water was added thereto to make the total volume of 20*µ*l. The mixture was incubated for 12 hours at 16°C. The expression vector containing an rhIFN-α gene so obtained was designated as Luck-α-IFN-1Y. The above procedures were shown in Fig. 3A.

### (4-B): Preparation of expression vector pYLBC-AG885-α-IFN

### <Step 1>: Synthesis of primer and PCR

UAS of promoter ADH2(5'-TCTCCAACTTATAAGTTGGAGA-3'), TATA box of promoter GAPDH and 111bp of DNA fragment containing a nucleotide sequence at the 5'-end of hIFN-α gene were subjected to PCR by using two primers ADHGAP and GAPIFN as follows.

Primer ADHGAP(5'-AGACGGATCCTCCTCTGCCGGAACACCGGGCATCTCCA
ACTTATAATGTTGGAGAAATAAGAGAATTTCAGATTGAGAGAATGAAAAAAAAAAACTGA
AAAAAAAGGTTGAAAC-3') and primer GAPIFN(5'-TTCTTCTAGAACCCAGGCT
GTGAGTTTGAGGCAGATCACACATGGTGTTTGTTTATGTGTG-3') were
synthesized.

Primer ADHGAP comprises 114 bases; and the 94 bases at the 5'-end contain a recognition site of BamHI and UAS of promoter ADH2; and the remainder comprises a nucleotide sequence which is capable of being linked to promoter GAPDH (-198th base to -178th base of GAPDH).

Primer GAPIFN contains a recognition site of restriction endonuclease XbaI, an initiation codon for the hIFN-α gene and a nucleotide sequence of promoter GAPDH comprising 18 bases(from -1st to -18th; Holland, et al., J. Biol. Chem. 254, 9839-9845(1979); Bitter et al., Gene 32, 263-274(1984)) at the 3'-end.

The structure of the primers is as follows.

Primer ADHGAP

5'-AGAC-GGATCC-TCCTCTGCCGGAACACCGGGCATCTCCAACTTATAATGTTGGAGAA

BamHI site          a base sequence containing UAS of
ATAAGAGAATTTCAGATTGAGAGAATGAAAAAAAAAAACT-GAAAAAAAAGGTTGAAAC-3'
promoter ADH2          GAPDH(-198th to -178th)

Primer GAPIFN

5'-TTCT-TCTAGA-ACCCAGGCTGTGAATTTGAGGCAGATCACACAT-

XbaI site rhIFN-α gene initiation codon

GGTGTTTGTTTATGTGTG-3'

a region of promoter GAPDH(-1st to -18th)

Using said two primers and a plasmid pYLBC5 containing the promoter GAPDH(ATCC 74119, see Korean Patent Appln. No. 91-25882) as a template, PCR was carried out as follows.

To a mixture of 10ng to 100ng of plasmid pYLBC5 DNA as a template DNA, 10*µ*l of 10 x Taq polymerase buffer(10mM Tris-HCl, pH 8.3, 500mM KCl, 15mM MgCl₂, 0.1%(w/v) gelatin), 10*µ*l of dNTP's mixture(1.25mM dGTP, dATP, dTTP and dCTP), 2*µ*g of a primer ADHGAP, 2*µ*g of a primer GAPIFN and 0.5*µ*l of Ampli Taq DNA polymerase(Perkin Elmer Cetus, U.S.A.) was added distilled water to make a total volume of 100*µ*l; and 50*µ*l of mineral oil was added thereto to prevent evaporation thereof. A first PCR was carried out by using thermocycler(Perkin Elmer Cetus, U.S.A.) and repeating 25 times the thermal cycle of: 95°C for 1 min → 55°C for 1 min; 72°C for 2 mins; and finally at 72°C for 10 mins.

To the reaction mixture was added an equal volume of phenol/chloroform; and the mixture was centrifuged to remove polymerase. The supernatant thereof was transferred to a new tube; and 1/10 volume of 3M sodium acetate, and 2.5 volumes of 100% ethanol were added thereto. The mixture was centrifuged to obtain about 320 bp of DNA fragment which was named AG885. The nucleic acid was dissolved in 20*µ*l of TE buffer (10mM Tris-HCl, pH 7.5, 1mM EDTA) for use in the next step.

### <Step 2>: Digestion of plasmid pYLBC5

2*µ*g of plasmid pYLBC5 was completely digested with the restriction endonucleases PstI and SalI in NEB buffer solution 3; and another 2*µ*g of plasmid pYLBC5 was also completely digested in the same manner. Each of the digested plasmids was subjected to 0.7% agarose gel electrophoresis to isolate about 9.84kb and 3.0kb fragments which were referred to as fragments PK and PS, respectively.

### <Step 3>: Isolation of rhIFN-α gene fragment

About 5*µ*g of vector M13mp18-IFN-α containing the rhIFN-α gene obtained in Example 3 was completely digested with the restriction endonucleases XbaI and SalI in NEB buffer solution 3 and subjected to agarose gel electrophoresis as to obtain about 450 bp of rhIFN-α gene fragment which was referred to as IFN-XL.

About 2*µ*g of fragment AG885 obtained in <Step 1> above was completely digested with the restriction endonucleases XbaI and BamHI in NEB buffer solution 3 and subjected to phenol/chloroform extraction and ethanol precipitation. The precipitated nucleic acid segment(hereinafter referred to as AG885-X/B) was dissolved in 10*µ*l of TE solution.

### <Step 4>: Preparation of plasmid pYLBC-AG885-α-IFN

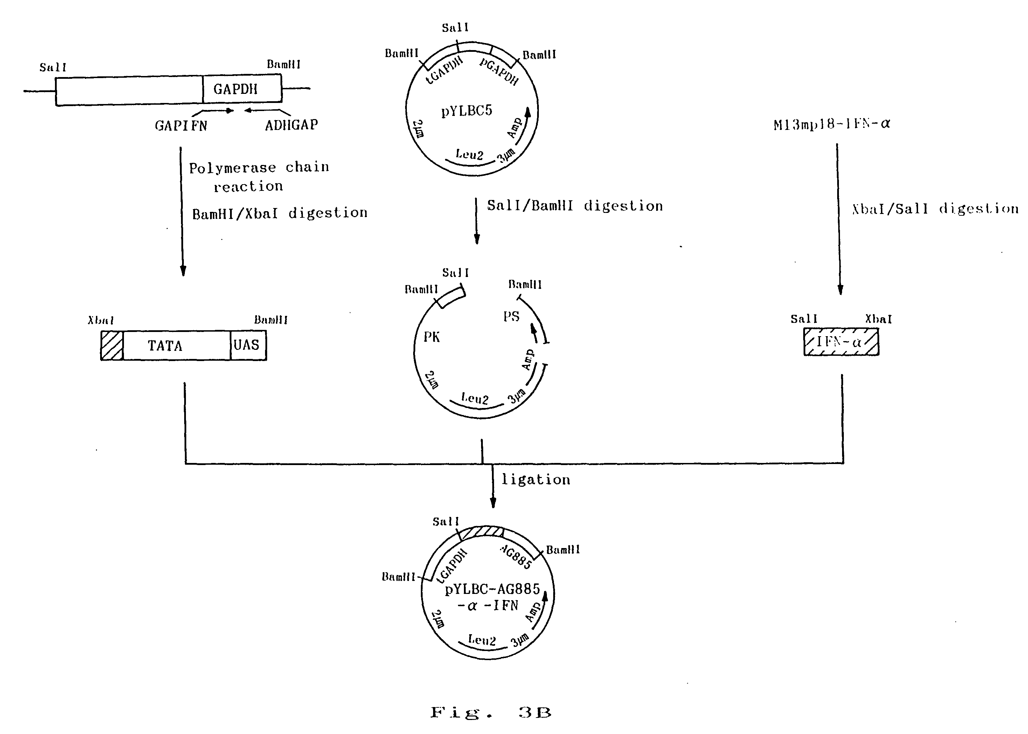

The fragments obtained in the above <Step 2> and <Step 3> were ligated as follows. To a tube were added 200ng of fragment IFN-X/L, 200ng of fragment AG885-X/B, 100ng of each of fragments PK and PS, 2*µ*l of 10 x ligation buffer and 10 units of T4 DNA ligase; and distilled water was added thereto to adjust the total volume to 20*µ*l. The mixture was reacted at 16°C for 12 hours. The above procedure is shown in Fig 3B.

### Example 5: Transformation of yeast with expression vector and expression of rhIFN-α gene

### <5-A>: Transformation of yeast with Luck-α-IFN-1Y and expression of rhIFN-α gene

The recombinant plasmid Luck-α-IFN-Y obtined in Example (4-A) was extracted in mass by means of alkalinelysis(Ish-Horowicz, D., et al., Nucl. Acid Res. 9, 2989(1981)) for use in the following transformation procedure. The transformation of yeast with plasmid was carried by using on the methods disclosed by Veges(Nature 275, 104(1978) and Hinnen (P.N.A.S. 75, 1929(1978)).

1ml of S. cerevisiae DCO4(Yeast Genetic Stock Center, Department of Biophysics and Medical Physics, University of California, CA 94720, U.S.A.) which had been cultured overnight was inoculated into 50ml of fresh YEPD medium and cultured with shaking at 30°C until the O.D. value at 650nm reached 0.8 to 1.0. The culture was centrifuged at 5000rpm for 5 minutes to isolate the cells. The precipitated cells were suspended in 20ml of water; and the suspension was recentrifuged. The cell precipitates were suspended in 20ml of 1M sorbitol solution; and the suspension was recentrifuged.

The precipitated cells were dissolved in 5ml of SP(1M sorbitol, 50mM sodium phosphate, pH 7.5) and 5*µ*l of 1M DTT; and 50*µ*l of xymolase(10mg/ml in 50% glycerol/50% sp) was added thereto, followed by culturing with shaking at 150rpm and 30°C for 30 minutes. During the cultivation, a small amount of the culture was occasionally taken and treated with 1/10 volume of 0.1% SDS to examine the degree of spheroplast formation with an optical microscope. The cultivation was carried out(generally for 30 minutes) until at least about 90% of the yeast cells were turned into spheroplast. After the completion of the cultivation, the culture was centrifuged at 1500rpm for 3 minutes with Dynac Centrifuge to obtain spheroplasts. The spheroplasts thus obtained were washed with 5ml of 1M sorbitol and 5ml of STC(1M sorbitol, 10mM CaCl₂, 10mM Tris-HCl, pH 7.5) and dissolved in 1ml of STC before use in its transformation with Luck-α-IFN-1Y.

To a mixture of 12.5*µ*l of 5 to 10*µ*g plasmid nucleic acid and 12.5*µ*l of 2 x STC was added 50*µ*l of the spheroplast, followed by the culturing thereof for 10 minutes at room temperature. To the culture was added 500*µ*l of PEG/TC solution(40% PEG-4000, 10mM CaCl₂, 10mM Tris-HCl, pH 7.5), which was stood for 10minutes. The culture was centrifuged at 1500rpm for 13 minutes with Dynac Centrifuge to isolate spheroplast. The precipitated spheroplast was dissolved in 200*µ*l of 1M sorbitol solution.

The solution containing the spheroplast was mixed with 7ml of a regeneration agar(3g of Bacto agar, 50ml of 2M sorbitol, 0.67g of yeast nitrogen base without amino acid, 0.1g of mixture of amino acids without leucine, 0.4ml of 50% glucose, 45ml of water per 100ml); and the mixture was poured onto a leuicine-deficient plate(182g of sorbitol, 20g of Bacto agar, 6.7g of yeast nitrogen base without amino acid(Difco, U.S.A.), 0.25g of Leu⁻ supplement, 4ml of 5% threonine, 40ml of 50% glucose and 820ml of water) and cultured at 30°C for 3 to 5 days to obtain yeast colonies transformed with the recombinant plasmid Luck-IFN-α-1Y.

A transformed yeast colony was cultured in 3ml of a leucine-deficient medium(6.7g of yeast nitrogen base without amino acid, 0.25g of mixture of amino acids without leucine and 6% glucose per 1L) at 30°C for 24 hours; and then the culture was inoculated into 100ml of YEPD medium(2% peptone, 1% yeast extract, 2% glucose), which was cultured at 30°C for 24 hours.

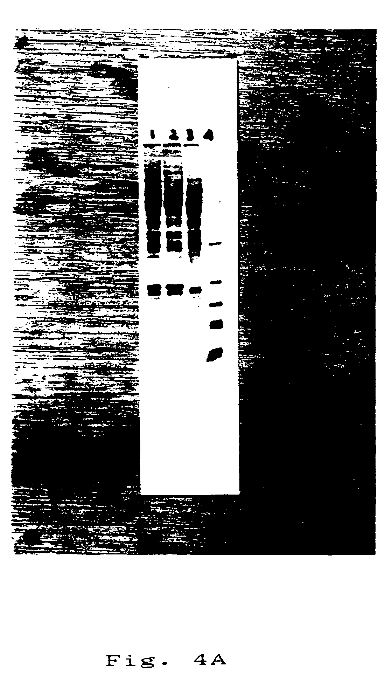

The final O.D. value of the culture was about 25 at 650nm. The amount of the culture corresponding to an O.D. value of 10 was centrifuged and dissolved in 400µl of a buffer(10mM Tris-HCl, pH 7.5, 1mM EDTA, 2mM PMSF, 8M urea). To the solution was added an equal volume of glass beads having a diameter of 0.4mm followed by vigorous stirring to disrupt the cell wall. 10*µ* l of the yeast extract thus obtained was subjected to electrophoresis on 15% SDS-polyacrylamide gel in accordance with the method disclosed by Laemmli et al. in Nature 277, 680(1970) and stained with Coomassie Brilliant Blue R250 to confirm the expression of the gene product. The result is shown in Fig. 4A.

In Fig. 4A, both of lanes 1 and 2 describe extract of yeast cell containing the expression vector Luck-α-IFN-1Y; lane 3 shows extract of yeast cell which does not contain the expression vector; and lane 4 shows a standard protein size marker which represents the molecular weights of 43,000, 29,000, 18,400, 14,300, 6,500 and 3,800 dalton from the top, respectively.

### <5-B>: Transformation of veast with expression vector pYLBC-AG885-α-IFN and expression of rhIFN-α gene

The recombinant plasmid pYLBC-AG885-α-IFN obtined in Example(4-B) was prepared in mass by means of alkaline lysis method(Ish-Horowicz, D. et al., Nucl. Acid Res. 9, 2989(1981)) for use in the transformation.

The transformation procedure of Example (5-A) was repeated by employing the plasmid pYLBC-AG885-α-IFN instead of the plasmid Luck-α-IFN-1Y to obtain yeast colonies transformed with the recombinant plasmid pYLBC-AG885-α-IFN.

The transformed yeast was cultured in 3ml of a leucine-deficient medium(6.7g of yeast nitrogen base without amino acid, 0.25g of mixture of amino acids without leucine and 6% glucose per 1L) at 30°C for 24 hours; and then the culture was inoculated into 100ml of YEPD medium(2% peptone, 1% yeast extract, 2% glucose) and cultured at 30°C for 24 hours.

The final O.D. value of the culture was about 25 at 650nm. The amount of the culture corresponding to the O.D. value of 10 was centrifuged and dissolved in 400*µ*l of a buffer(10mM Tris-HCl, pH 7.5, 1mM EDTA, 2mM PMSF, 8M urea). To the solution was added an equal volume of glass beads having a diameter of 0.4mm followed by vigorous stirring to disrupt the cell wall. 10*µ* l of the yeast extract thus obtained was subjected to electrophoresis on 15% SDS-polyacrylamide gel in accordance with the method disclosed by Laemmli et al. in Nature 277, 680(1970) and stained with Coomassie Brilliant Blue R250 to confirm the expression of the gene product. The result is shown in Fig. 4B.

In Fig. 4B, lane 1 shows extract of yeast cell which does not contain the expression vector pYLBC-AG885-α-IFN, lanes 2 and 3 show extract of yeast cell containing the expression vector pYLBC-AG885-α-IFN after its cultivation for 4 hours; lanes 4 and 5 show extract of yeast cell containing the expression vector pYLBC-AG885-α-IFN after its cultivation for 48 hours, respectively; and lane 6 shows the standard protein size marker which represents the molecular weights of of 43,000, 29,000, 18,400, 14,300, 6,500 and 3,500 dalton from the top, respectively.

### Example 6: (for demonstrative purposes only; not claimed as such) Purification of hIFN-α produced in yeast

### (6-A): Disruption of culture of yeast transformants

1kg of yeast transformed with the recombinant plasmid Luck-α-IFN-1Y obtained in Example(5-B), in which hIFN-α was expressed, was suspended in a buffer(20mM Tris-HCl, 1M urea, 1mM EDTA, 1mM PMSF, pH 8.0) using homogenizer(Beckman) and disrupted at a speed of 750ml/min with Bead Beater(Biospec Product, U.S.A) packed with glass beads having a diameter of 0.5 to 0.75mm. The solution containing the disrupted yeast was centrifuged at 12,000rpm and 4°C for 50 minutes with a centrifuge(Backman JA-14, Rotor). The supernatant was discarded and the precipitates were recovered. The collected precipitates were suspended in 2L of a buffer(20mM Tris-HCl, 1M urea, 1mM EDTA, 0.1% Triton X-100, pH 8.0) using a homogenizer(Beckman); and the suspension was centrifuged at 12,000rpm for 50 minutes with a centrifuge (Beckman JA-14 Rotor) to isolate the precipitates. The collected precipitates were further suspended in 2L of 1M guanidine solution(guanidine HCl, Amresco) using a homogenizer; and the suspension was centrifuged at 12,000rpm for 30 minutes to isolate about 600g of the precipitates.

### (6-B): Dissolution of the protein precipitates in a guanidine solution

600g of the precipitates obtained in Example(6-A) was dissolved in 1.8L of 6M guanidine solution(6M guanidine-HCl, 20mM Tris-HCl, 100mM β-mercaptoethanol, pH 8.0) at 4°C overnight with stirring. The solution was centrifuged at 12,000rpm and 4°C for 30 minutes with a centrifuge(Beckman, JA-14 Rotor) to remove the precipitate. About 2.1L of the collected dark brownish supernatant was 4-fold diluted with a buffer solution(20mM Tris-HCl, 0.1mM PMSF, pH 8.0) and centrifuged to recover the supernatant. The supernatant was concentrated with an ultrafiltration membrane to a volume of 2L and then the concentrate was dialyzed three times to 50L of a buffer solution(20mM Tris-HCl, 0.12M NaCl, 0.1mM PMSF, pH 8.0) at 4°C.

### (6-C): DEAE-cellulose column chromatography

The solution obtained in Example(6-B) was centrifuged at 12,000rpm and 4°C for 30 minutes with a centrifuge(Beckman, JA-14 Rotor) to remove the precipitates. The collected supernatant was loaded on a DEAE-cellulose resin column(Whatman) equilibrated with a buffer solution(20mM Tris-HCl, 0.12M NaCl, 0.1mM PMSF, pH 8.0) at a rate of 10cm/hr. The protein solution passed through the column without binding to the resin was dialyzed three times with 50L of a buffer solution(50mM sodium acetate, pH 4.5).

The dialyzed protein solution was centrifuged at 12,000rpm and 4°C for 30 minutes with a centrifuge(Beckman, JA-14 Rotor) to remove the precipitates.

### (6-D): CM-Sephalose column chromatography

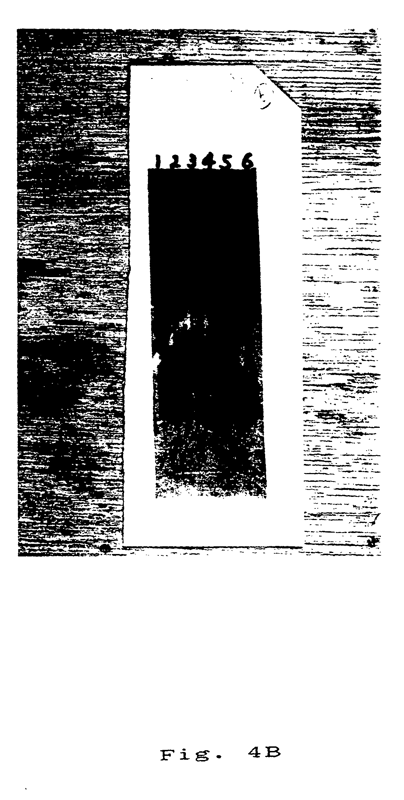

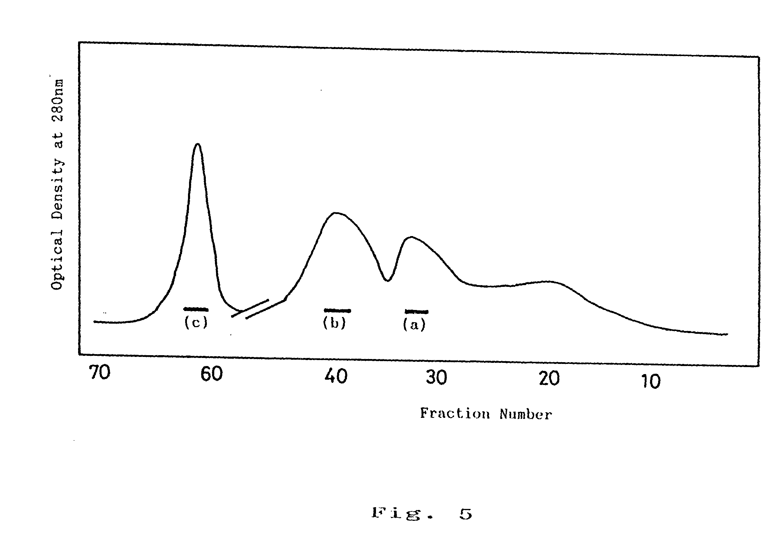

A mixture of 50mM sodium acetate and the dialyzed protein solution(pH 4.5) obtained in Examle(4-C) was loaded on a CM-Sepharose resin column equilibrated with a buffer solution (20mM Tris-HCl, 0.12M NaCl, 0.1mM PMSF, pH 8.0) at a rate of 15cm/hr . The column was washed with the same buffer solution to discharge the proteins not binding to the resin until the peak of the proteins dropped sufficiently. Thereafter, an elution of protein was carried out using a buffer solution(50mM sodium acetate, 0.12M sodium chloride, pH 4.5). When the peak dropped, another elution was further carried out using a buffer solution(50mM sodium acetate, 0.5M sodium chloride, pH 4.5). The result of CM-Sepharose chromatography is shown in Fig. 5 as the O.D. value at 280nm.

In Fig. 5, dark bar(a) indicates fractions containing a partially oxidized hIFN-α; dark bar(b) indicates fractions containing mainly active form of monomeric hIFN-α which is substantially identical to natural hIFN-α; and dark bar(c) indicates fractions containing mainly dimeric hIFN-α. Partially oxidized hIFN-α, active form of hIFN-α and dimeric hIFN-α were eluted in order. In order to completely remove the partially oxidized hIFN-α and dimeric hIFN-α from hIFN-α, the solution of fractions corresponding to the bar(b) was subjected to CM-Sepharose column chromatography, thereby obtaining highly purified active form of monomeric hIFN-α.

If necessary, further purfication by gel permeation chromatography may be carried out.

The protein solution obtained above was passed at a flow rate of 3cm/hr through an S-300(Pharmacia) column equilibrated with a buffer solution(20mM NH₄-acetate, 0.12M NaCl, pH 4.5). The eluted fractions were subjected to non-reducing SDS-PAGE and the only fractions containing active form of monomeric hIFN-α and not substantially containing oligomeric hIFN-α were collected. The collected protein solution was dialyzed three times to 20L of PBS buffer solution.

### (6-E): Conversion of hIFN-α in inactive form to active form

Dimeric hIFN-α obtained in Example(6-D) ((c) of Fig. 5) which was identified by 15% non-reducing SDS-PAGE was collected and dialyzed to 20mM Tris-HCl(pH 8.0).

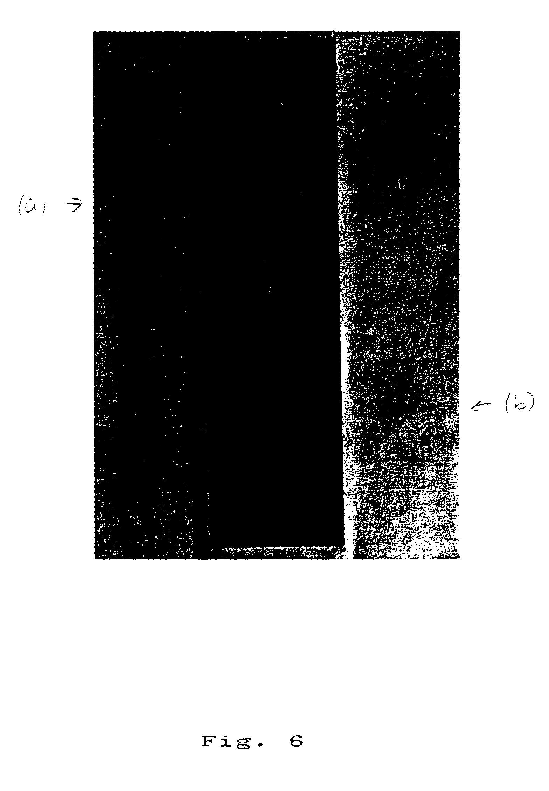

To the above dialyzed solution were added cysteine(Sigma) and cystine(Sigma) to a concentration of 5mM and 0.5mM, respectively, followed by stirring and standing over 5 hours at 4°C or at room temperature; the solution was subjected to SDS-polyacrylamide gel electrophoresis to confirm that the dimer was converted into the monomer; and, thereafter, the sample was dialyzed to a buffer solution(50mM sodium acetate, pH4.5). Fig. 6 shows the results of 15% non-reducing SDS-PAGE for the dimeric hIFN-α before the above conversion(a) and the monomeric hIFN-α coverted above(b).

The same result was also obtained by using an oxidized glutathione and a reduced glutathione.

The dialyzed sample was subjected to CM-Sepharose column chromatography and gel permeation chromatography by the same procedure as in Example(6-D) to obtain highly purified active form of monomeric hIFN-α.

The partially oxidized hIFN-α obtained in Example(6-D) ((a) of Fig. 5) was subjected to the same conversion steps as above to obtain an active form of hIFN-α.

### (6-F): Determination of bioactivity of hIFN-α

The bioactivity of the purified hIFN-α was determined by measuring the anti-cytopathic activity of hIFN-α against virus as follows.

A suspension of male calf kidney cell(MDBK, 3.5 x 10⁵ cells/ml) which was cultured in T75 flask was spread onto 96 blank plates in an amount of 100*µ*l, respectively, and cultured in 5% CO₂ incubator at 37°C for 18 to 24 hrs. 100*µ*l of vesicular stomatitis virus(1 x 10⁵ to 4 x 10⁵ pfu/ml) was inoculated to wells washed with PBS buffer(pH 7.0), and cultured for 18 to 24 hrs in 5% CO₂ incubator at 37°C to form a single layer of cells. 100*µ*l of each of the hIFN-α samples obtained in Examples (6-D) and (6-E) was two-fold diluted serially, added to each well and left for 48hrs in 5% CO₂ incubator at 37°C. Thereafter, a crystal violet dye solution was added to each well and the O.D. value at 600nm was measured.

The protein concentration of the hIFN-α was determined by means of Lowry's method. A standard hIFN-α, Gxa-01-901-535, was obtained from NIH of the U.S. and the specific activity of which was 2.0 x 10⁸ IU/mg.

The result of activity determination indicated that both of monomeric hIFN-αs obtained in Examples (6-D) and (6-E) had a specific activity of 1.8 x 10⁸ IU/mg or more. Accordingly, the hIFN-α converted from the dimeric hIFN-α and patially oxidized hIFN-α was equivalent to the active form of monomeric hIFN-α obtained initially by CM-Sepharose chromatography in terms of their biological activity.

### (6-G): Reverse phase HPLC

A reverse phase HPLC was carried out to confirm the identity between the hIFN-αs obtained in Examples (6-D) and (6-E).

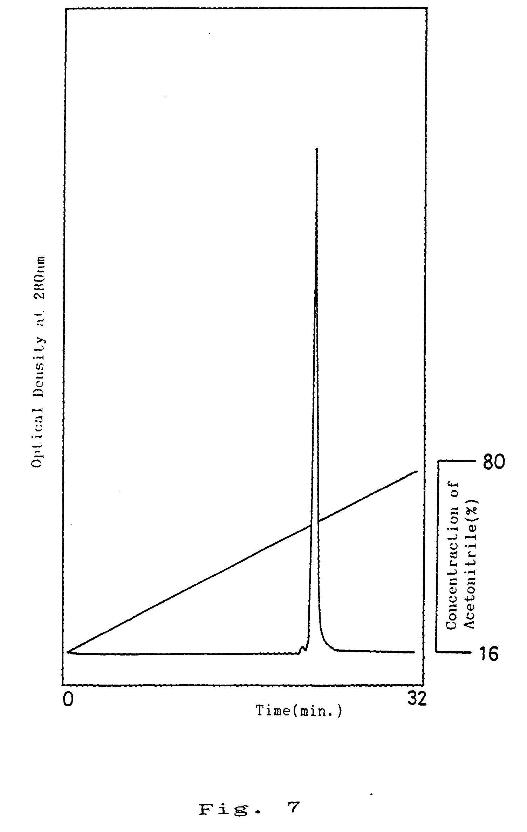

Two protein samples were passed through a C18 column(*µ*-Bondapak, Waters) equilibrated with 15%(v/v) acetonitrile(15% acetonitrile/0.05% trifluoroacetic acid/H₂O) with a rapid linear gradient of 25%(v/v) acetonitrile over 5 minutes and then a gentle linear gradient of 56%(v/v) acetenitrile over 35 minutes. The result is shown in Fig. 7 wherein two proteins were eluted at the same position. Accordingly, it can be construed that said hIFN-αs obtained by the above two methods are identical in terms of physiochemistry.

While the invention has been described with respect to the above specific embodiments, it should be recognized that various modifications and changes which may be apparent to those skilled in the art to which the invention pertains may be made and also fall within the scope of the invention as defined by the claims that follow.

## Claims

1. A recombinant human alpha interferon gene having the following nucletoide sequence:

30
ATG TGT GAT CTG CCT CAA ACT CAC AGC CTG
60
GGT TCT AGA AGA ACC TTG ATG TTG CTA GCT
90
CAA ATG AGA AAG ATA AGC TTG TTC TCC TGC
120
TTG AAG GAC AGA CAT GAC TTT GGA TTT CCC
150
CAG GAG GAG TTT GGC AAC CAG TTC CAA AAG
180
GCT GAA ACC ATC CCT GTC CTC CAT GAG ATG
210
ATC CAG CAG ATC TTC AAT CTC TTC AGC ACA
240
AAG GAC TCA TCT GCT GCT TGG GAT GAG ACC
270
CTC CTA GAC AAA TTC TAC ACT GAG CTC TAC
300
CAG CAA CTG AAT GAC CTG GAA GCC TGT GTG
330
ATA CAG GGG GTG GGG GTG ACA GAG ACT CCC
360
CTG ATG AAG GAG GAC TCC ATT CTG GCT GTG
390
AGG AAA TAC TTC CAA AGA ATC ACT CTC TAT
420
CTG AAA GAG AAG AAA TAC AGC CCT TGT GCC
450
TGG GAG GTT GTC AGA GCA GAA ATC ATG AGA
480
TCC TTT TCT TTG TCA ACA AAC TTG CAA GAA
AGT TTA AGA AGT AAG GAA TAA TAG

2. An expression vector comprising the recombinant human alpha interferon gene of claim 1, wherein the gene is positioned at a site capable of being expressed in a yeast cell.

3. The vector of claim 2 which is Luck-α-IFN-1Y(KCCM 10019).

4. The vector of claim 2 which is pYLBC-AG885-a-IFN(KCTC 0028BP).

5. A yeast cell transformed with the expression vector of claim 2.

6. The transformed yeast cell of claim 5 which is Saccaromyces cerevisiae DC04 Luck-α-IFN-1Y.

7. The transformed yeast cell of claim 5 which is Saccaromyces cerevisiae DC04 pYLBC-AG885-α-IFN.

8. A process for producing human alpha interferon comprising culturing the yeast cell of claim 5, 6 or 7.

## Patentansprüche

1. Rekombinantes Human-alpha-Interferon-Gen mit der folgenden Nucleotidsequenz:

30
ATG TGT GAT CTG CCT CAA ACT CAC AGC CTG
60
GGT TCT AGA AGA ACC TTG ATG TTG CTA GCT
90
CAA ATG AGA AAG ATA AGC TTG TTC TCC TGC
120
TTG AAG GAC AGA CAT GAC TTT GGA TTT CCC
150
CAG GAG GAG TTT GGC AAC CAG TTC CAA AAG
180
GCT GAA ACC ATC CCT GTC CTC CAT GAG ATG
210
ATC CAG CAG ATC TTC AAT CTC TTC AGC ACA
240
AAG GAC TCA TCT GCT GCT TGG GAT GAG ACC
270
CTC CTA GAC AAA TTC TAC ACT GAG CTC TAC
300
CAG CAA CTG AAT GAC CTG GAA GCC TGT GTG
330
ATA CAG GGG GTG GGG GTG ACA GAG ACT CCC
360
CTG ATG AAG GAG GAC TCC ATT CTG GCT GTG
390
AGG AAA TAC TTC CAA AGA ATC ACT CTC TAT
420
CTG AAA GAG AAG AAA TAC AGC CCT TGT GCC
450
TGG GAG GTT GTC AGA GCA GAA ATC ATG AGA
480
TCC TTT TCT TTG TCA ACA AAC TTG CAA GAA
AGT TTA AGA AGT AAG GAA TAA TAG

2. Expressionsvektor, umfassend das rekombinante Human-alpha-Interferon-Gen von Anspruch 1, wobei das Gen an einer Stelle, die exprimiert werden kann, in einer Hefezelle angeordnet ist.

3. Vektor nach Anspruch 2, der Luck-α-IFN-1Y (KCCM 10019) ist.

4. Vektor nach Anspruch 2, der pYLBC-AG885-α-IFN (KCTC 0028BP) ist.

5. Hefezelle, die mit dem Expressionsvektor von Anspruch 2 transformiert ist.

6. Transformierte Hefezelle nach Anspruch 5, die Saccaromyces cerevisiae DC04 Luck-α-IFN-1Y ist.

7. Transformierte Hefezelle nach Anspruch 5, die Saccaromyces cerevisiae DC04 pYLBC-AG885-α-IFN ist.

8. Verfahren zur Herstellung von Human-alpha-Interferon, umfassend das Kultivieren der Hefezelle von Anspruch 5, 6 oder 7.

## Revendications

1. Gène recombinant d'interféron alpha humain ayant la séquence nucléotidique suivante:

30
ATG TGT GAT CTG CCT CAA ACT CAC AGC CTG
60
GGT TCT AGA AGA ACC TTG ATG TTG CTA GCT
90
CAA ATG AGA AAG ATA AGC TTG TTC TCC TGC
120
TTG AAG GAC AGA CAT GAC TTT GGA TTT CCC
150
CAG GAG GAG TTT GGC AAC CAG TTC CAA AAG
180
GCT GAA ACC ATC CCT GTC CTC CAT GAG ATG
210
ATC CAG CAG ATC TTC AAT CTC TTC AGC ACA
240
AAG GAC TCA TCT GCT GCT TGG GAT GAG ACC
270
CTC CTA GAC AAA TTC TAC ACT GAG CTC TAC
300
CAG CAA CTG AAT GAC CTG GAA GCC TGT GTG
330
ATA CAG GGG GTG GGG GTG ACA GAG ACT CCC
360
CTG ATG AAG GAG GAC TCC ATT CTG GCT GTG
390
AGG AAA TAC TTC CAA AGA ATC ACT CTC TAT
420
CTG AAA GAG AAG AAA TAC AGC CCT TGT GCC
450
TGG GAG GTT GTC AGA GCA GAA ATC ATG AGA
480
TCC TTT TCT TTG TCA ACA AAC TTG CAA GAA
AGT TTA AGA AGT AAG GAA TAA TAG

2. Vecteur d'expression comprenant le gène recombinant de l'interféron alpha humain selon la revendication 1, dans lequel le gène est positionné dans un site susceptible d'être exprimé dans une cellule de levure.

3. Vecteur selon la revendication 2 qui est le Luck-α-IFN-1Y (KCCM 10019)

4. Vecteur selon la revendication 2 qui est le pYLBC-AG885-α-IFN (KCTC 0028BP).

5. Cellule de levure transformée avec le vecteur d'expression selon la revendication 2.

6. Cellule de levure transformée selon la revendication 5 qui est Saccharomyces cerevisiae DC04 Luck-α-IFN-1Y.

7. Cellule de levure transformée selon la revendication 5 qui est Saccharomyces cerevisiae DC04 pYLBC-AG885-α-IFN.

8. Procédé de production d'interféron alpha humain comprenant la culture de cellules de levure selon les revendications 5, 6 ou 7.
